# EUROPEAN PATENT APPLICATION

(11) **EP 1 398 375 A1**
(43) Date of publication of application: **17.03.2004**
(21) Application number: 02021514.1
(22) Date of filing: 26.09.2002
(51) Int. Cl.: C12N 15/10, C12Q 1/68

(54) **Recombination process for recombining fragments of variant polynucleotides**

(30) Priority: 13.09.2002 EP 02020639
(71) Applicant: Cosmix Molecular Biologicals GmbH, 38124 Braunschweig (DE)
(72) Inventor: Collins, John, Prof. Dr., 38100 Braunschweig (DE); Derer, Jutta, Dr., 38102 Braunschweig (DE); Szardenings, Michael, Dr., 38302 Wolfenbuettel (DE); Lütjohann, Jens, Dr., 22763 Hamburg (DE)
(74) Representative: König, Gregor Sebastian, Dipl.-Biol.

(57) **Abstract**

The invention relates to an improved recombination process wherein at least fragments of variant polynucleotides are recombined, wherein at least fragments of the polynucleotides are amplified by the use of at least one oligonucleotide primer which comprises a 5' sequence section carrying a recognition site for a PRE and which primes at the desired recombination site, wherein the amplification products are cleaved with at least one PRE recognising the recognition site introduced by the at least one oligonucleotide primer thus creating a recombination site, and wherein the cleaved fragments are ligated thus producing recombinant molecules. The present invention allows the recombination of polynucleotide sequences and/or fragments thereof in a controlled fashion in vitro. In contrast to existing technologies, neither a high sequence identity or similarity between the molecules undergoing recombination is required, nor must the restriction enzyme binding site be present within the polynucleotide sequences undergoing recombination.

## Description

The present invention relates to an improved recombination method which allows the recombination of polynucleotides which share little or no homology at the recombination site. Further an improved method for the generation of molecules with desired traits or characteristics is provided.

Biotech evolutionary methods are used to search for novel ligands or molecules such as polynucleotides or polypeptides using empirical procedures to select molecules with the desired characteristics, e.g. binding properties of polypeptides, from large populations of variant gene products. These methods are comparable to the process of natural evolution, since evolution includes the generation of mutation, selection of functionality over a time period and the ability of the system to self-replicate.

In order to create molecular diversity in a library in order to generate mutant molecules several methods are known in the state of the art as for example the error-prone polymerase chain reaction and cassette mutagenesis, in which the specific region to be optimised is replaced with a synthetically mutagenised oligonucleotide. Error-prone PCR uses low-fidelity polymerisation conditions to introduce a low level of point mutations randomly over a long sequence. This method can be used to mutagenise a mixture of fragments of unknown sequence but is limited when applied for multiple cycles due to the fact that, for example, harmful or neutral mutations accumulate. Thus error-prone PCR can lead to undesired results and, beyond this, is too gradual to allow block changes that are required for continued sequence evolution.

Also oligonucleotide directed mutagenesis, with a short sequence being replaced by synthetically mutagenised oligonucleotides does not allow generation of combinations of distant mutations and is thus not significantly combinatorial. A high number of selection rounds and sequencing is necessary and the process thus being labour- and cost-intensive and not practicable for multiple rounds of mutagenesis.

The introduction of base changes in existing (also mutant) structures is an additive and thus a linearly increasing function. A process which relies on mutagenesis, selection and further mutagenesis also has the drawback that potentially synergistic mutations in different regions are lost since they are not selected in combination. Thus recombination processes are advantageous over processes which rely on mutagenesis in a large library. The considerable amount of extra work involved in trying to optimise a sub-optimal affinity of a lead peptide or protein, e.g. as isolated from even large libraries, but without taking the advantage of recursive or reiterative recombination or mutagenesis and recombination has been addressed before (Collins, 1997; Deshayes et al., 2002).

Recombination processes which reassort mutations accumulated in the selected population lead to an exponential increase of the combinations of mutations and thus to an increase of the number of variants in a population. Several in vitro methods are known in the state of the art which allow the production of recombinant molecules which can be analysed and selected for desired characteristics afterwards.

Most in vitro approaches known in the state of the art are based on PCR and on homologous recombination. These methods are variously referred to "as sexual PCR" or "DNA shuffling", methods which aim at mimicking and accelerating evolution in the test tube to develop proteins with novel or improved characteristics (see for example EP 1 103 606 A1; EP 0911 396 A1; EP 1 138 763 A1; Stemmer et al., 1995; Crameri and Stemmer, 1995; Schmidt-Dannert et al., 2000; Joern et al., 2002). This latter line of pursuit is often combined with reiterative rounds of mutation, recombination and selection. The initial procedures described, involve randomized partial cleavage of populations of DNA molecules with unspecific endonucleases such as DNAsel and DNA amplification of the fragments without the addition of specific primers (as is usual with PCR) to reconstitute the whole-length gene. The generated fragments are suitable for PCR amplification which is performed in order to recombine the fragments, yielding a shuffled pool of recombined polynucleotides. The fragments can anneal with each other thus leading to cross-over reactions, since a DNA fragment derived from one template primes DNA synthesis by binding on the homologous position of a related but different template. The products are subsequently cloned as a cleaved unit length molecule into an appropriate expression vector and screened for desired properties in an appropriate host organism.

Further variants of DNA shuffling which all use PCR as a central feature for recombining DNA molecules have also been described, as for example the staggered extension process (Zhao et al., 1998), random priming recombination (US 5,565,408), DNA reassembly by interrupted synthesis (Short, 1999) and restriction based shuffling (Kikuchi et al., 1999). All those methods differ from each other in the procedure to generate fragments in order to reassemble by sexual PCR. These methods depend on a stochastic process that reassembles genes based on homology.

The mentioned recombination methods have various limitations. If they are carried out within a population of homologous DNA molecules, having around 99 to ca. 70% sequence homology (see e.g. Joern et al., 2002, where heptamer homology core sequences with variable G+C content are considered essential) recombination will occur. But crossovers are only generated in regions of the highest sequence identity. Groups which have studied the bias in recombination sites used during DNA shuffling with DNA segments of low homology, pointed out that this methodology is not very suitable for this task since most of the molecules obtained were parental, i.e. had not undergone recombination, or had been preferentially involved in recombination at sites of highest homology (Schmidt-Dannert et al., 2000; Joern et al., 2002). Thus sexual PCR suffers severe linkage effects that make crossovers in regions of identity of < 15 bases rare. This effect was neither desired nor originally discussed or anticipated by the inventors of the mentioned methodology although it can be considered a major antithesis to obtaining the desired randomized recombination. If a disproportionate number of parental molecules remain in the population this will cause an inordinate increase in the amount (numbers of clones to be screened) of screening necessary to find advantageous recombinants. Furthermore the number of crossovers is also limited. A different in vitro method (Coco et al, 2001) allows the generation of more cross-overs which also occur in regions of short (less than 5 bases) homology. With this method it is not possible to control the position of the cross-over and it is quite labor intensive.

Thus sexual PCR and similar DNA-homology-dependent methods are not suitable for initiating recombination at sites which exhibit little or no homology nor are they suitable for the initiation of recombination at predetermined cross-over positions. However, there are many molecules having very similar three-dimensional structures, and even closely related functions, but which show low or no significant sequence similarity on the DNA level. Thus the desirable option to recombine polynucleotides with low or no homology on the DNA level is quite limited with sexual PCR, although, in fact, erroneously, this methodology has been portrayed previously as optimal for directed evolution using gene families of low homology (e.g. [Crameri et al., 1998]). Furthermore, the methods mentioned do not allow the block-wise assembly of new polynucleotides sequences out of fragments.

Further recombination methods are known in the State of the Art, which allow the recombination of molecules which share little or no homology. One method having these advantages is the SHIPREC method (Sieber et al, 2001). The key step in this method is the fusion of two parental genes in order to form a gene dimer which is digested with DNAseI to form an ensemble of random-length fragments. Fragments of a length corresponding to either of the parent sequences are isolated. After blunt end generation the remaining ends of the two genes are fused by circularization. The gene that was in the 5' position in the dimer will then be at the 3' position and will donate the C-terminus of the hybrid protein. This method has the draw back that it is quite work intensive due to the necessity to separate the fragments by size. Further many non-functional clones are created due to frameshifts in the cross over position leading to massive mutation and premature truncation of the proteins. The inventors report that actually two thirds of the library are not functional. Thus this method is inefficient and makes, disadvantageously, pre-selection procedures necessary.

A further method, not relying on sequence homologies, is ITCHY (Ostermeier et al, 1999), which is based on incremental truncation. This method can be used to create a library of fusions between all possible fragments of two genes. Overlapping fragments of two genes are cloned into suitable vectors for incremental truncation. The plasmid DNA is restricted with two restriction enzymes; one which produces a 3' recessed end (susceptible to ExoIII digestion) and the other that produces a 5' recessed end (resistant to ExoIII digestion). The fragments are digested with exonuclease III and aliquots are removed in intervals thus obtaining a library of deletions of all possible lengths from one end of the fragment. The ends of the DNA are blunted and upon ligation an incremental truncation library is created. Fusion is achieved by the use of a restriction site at the 3' end of the C-terminal gene. Since the ligation is a random process all possible fragments of one gene will be fused to all possible fragments of the other genes. This method has the drawback that due to the undirected truncation many rejects are present in the library since the parent gene length is not conserved and frameshifts occur.

It is an underlying problem of the invention to provide an efficient recombination method, which allows the recombination of polynucleotides which share little or no homology at the recombination site.

It is a further underlying problem of the invention to provide a process for evolving a molecule by recombination and selection, wherein recombination is also initiated at sites which have little or no homology.

The problems are solved by the independent claims. Preferred embodiments are defined in the dependant claims.

According to a first embodiment of the present invention a process for recombining at least fragments of variant polynucleotides is provided, wherein
- at least fragments of the polynucleotides are amplified by the use of at least one oligonucleotide primer which comprises a 5' sequence section carrying a recognition site for a PRE and which primes at the desired recombination site,
- the amplification products are cleaved with at least one PRE recognising the recognition site introduced by the at least one oligonucleotide primer thus creating a recombination site,
- the cleaved fragments are ligated thus producing recombinant molecules.

The present invention allows the recombination of polynucleotide sequences and/or fragments thereof in a controlled fashion in vitro. In contrast to existing technologies, neither a high sequence identity nor similarity between the molecules undergoing recombination is required, nor must the restriction enzyme binding site be present within the polynucleotide sequences undergoing recombination.

Polynucleotide releasing enzymes (PREs) comprise proteins possessing endonucleolytic activity, where the cleavage of a polynucleotide polymer takes place at a site physically removed, but at a fixed distance, measured as a specific number of nucleotide bases, from the specific recognition or binding site (lists of such enzymes and their properties can be accessed at http://rebase.neb.com). The first type of enzyme recognised as having such properties were the type IIs restriction endonucleases ( [Podhajska and Szybalski, 1985 ];[Szybalski, 1985]). The term PRE however, also encompasses chimerical protein genes encoding products which contain the unspecific cleavage domain of the endonuclease joined to a novel specific DNA binding site [Aggarwal and Wah, 1998].

Because of the feature that the polynucleotides or fragments thereof are amplified with at least one oligonucleotide primer per polynucleotide or polynucleotide fragment which carries a recognition site for a PRE enzyme at the 5' sequence section, at least one external PRE recognition site is attached adjacent to the recombination site of each polynucleotide or polynucleotide fragment intended to participate in the recombination process. Upon cleavage the attached PRE site is eliminated. The introduction of appropriate PRE recognition sites by use of the oligonucleotide primers has the advantage that it is not necessary to first analyse the polynucleotides which are to be recombined for appropriate restriction sites or to introduce restriction sites and thereby to (disadvantageously) alter the original sequence by e.g. site directed mutagenesis in case no appropriate sites are present. Furthermore with some recombination processes it is desirable that the PRE recognition site is not longer present in the recombined molecule. With the method according to the present invention a recombination site can literally be generated at any position of the polynucleotide since the PRE recognises the binding site attached by the oligonucleotide primer but cleaves adjacent within the polynucleotide sequence at the desired position if the spacing between the introduced PRE recognition site and the desired recombination site is chosen correctly. Thus a great freedom for creating crossover sites is generated since there is no restriction to existing restriction sites or homologous sequences. Also frameshifts as they occur with the methods known in the state of the art are avoided since the recombination site can be predetermined and chosen very precisely in order to avoid frameshifts. Further as many cross over sites as desired can be created at any desired position, a characteristic which is an advantage over the methods known in the state of the art.

Preferably the PRE recognition site introduced by the oligonucleotide primer is not present in the polynucleotide fragments to be recombined. Due to the fact that only little or even no homology is needed, the invention allows the ordered recombination of polynucleotides that are not highly related on the nucleotide level but where either the oligonucleotides can share homology on the secondary or tertiary level or the peptides or proteins encoded by these can share homology on the primary, secondary or tertiary level. In a further embodiment the ordered assembly of even completely unrelated polynucleotide fragments will be revealed in further detail below. The recombination method according to the present invention is very efficient for the creation of large libraries in order to allow high throughput screening (HTS), since only a small number of rejects are created and thus present in the library, even in the absence of a selection process to eliminate rejects, since the recombinant clones are more likely to be recombinants of a correct overall structure. The recombination process can be carried out with linear or circular substrates.

It is preferred to amplify the polynucleotides or fragments thereof by PCR wherein two oligonucleotide primers are used for the amplification of each polynucleotide or fragment thereof as it is known in the state of the art. Also both oligonucleotide primer can carry a recognition site for a PRE in its 5' section if desired. This embodiment is especially preferred when more than one cross-over is desired and more than two fragments or polynucleotides are supposed to recombine. This feature is also suitable for the recombination or assembly of fragments in order to yield new polynucleotides. It is possible to use the same set of oligonucleotide primers for the amplification of different polynucleotides and/or fragments thereof. The use of the same set of oligonucleotide primers is especially preferred in case variant polynucleotides or fragments thereof are recombined within a library which share sequence homology on the DNA level at least at the priming site. It is also possible to use degenerate oligonucleotide primers.

The oligonucleotide primers used in the present invention have so far been used for site directed mutagenesis (Stemmer and Morris, 1992; Murakami et al., 2002) and for manipulating genetic constructs in order to replace segments (US 2001/0031483; US 6,261,797). However they have not been used for recombination processes according to the present invention.

According to the invention it is also possible to initiate recombination in addition to the process outlined above by the use of PRE recognition sites already present in the library or sites introduced by site-directed mutagenesis if desired.

For the purposes of the present invention the expression "polynucleotide" or "polynucleotide fragment" comprises any nucleotide sequence of natural or synthetic origin, single or double stranded which can also comprise analogous chemical structures. "Variant" means a variation in at least one nucleotide.

It is advantageous to create a non-palindromic cohesive end at the recombination site. The cohesive end can preferably comprise an overhang of at least one nucleotide up to around 8bp depending on the desired ligation efficiency. However cohesive ends of any desired length can be produced by using respective linkers which attach to the enzymatically produced cohesive end. Most preferably the cohesive end comprises around 2 to 5bp and is generated by the PRE. This feature allows the ordered assembly of the different fragments since the formation of head- to-head or tall-to-tail ligated recombinants is avoided. Thus the efficiency of the library is greatly enhanced. If different cohesive ends are created on fragments which are not desired to recombine with each other the tailored construction of recombinant polynucleotides is possible. By using unique compatible cohesive ends it is ensured that only fragments which are desired to recombine can ligate to each other and thus assemble in an ordered fashion. Thus it is not necessary to separate fragments which are not desired to recombine with each other during ligation or to perform the cleavage and ligation consecutively. Thus the recombination process can be carried out with mixtures of unrelated subsets without the need for separation while increasing diversity. If necessary the polynucleotide sequence can be altered at the actual cleavage (recombination) site in order to ensure that the overhang is non-palindromic and/or that the cohesive ends of different groups/fragments are incompatible.

According to a specific embodiment of the present invention it is possible to recombine at least two variant polynucleotides which share no homology at all at the recombination site. It is also possible to assemble at least two polynucleotides or fragments, thereof, thus forming a new polynucleotide according to this embodiment. For amplification of the parental polynucleotides at least four oligonucleotide primers A, B, C and D are provided, wherein the primers A and B are used for the amplification of the first polynucleotide and the primers C and D for the amplification of the second polynucleotide. The oligonucleotide primers B and C prime at the chosen recombination site and comprise a 5' section carrying a recognition site for a PRE. The oligonucleotide primers A and D carry at least one recognition site for a restriction enzyme. The polynucleotides are amplified with the oligonucleotide primers. A linear fusion product comprising the amplification products is created, wherein the head of the first polynucleotide is fused to the tail of the second polynucleotide. The fusion product is cleaved with at least one PRE recognising the recognition site introduced by the polynucleotide primers B and C. The terminal fragments which were introduced by the oligonucleotide primers and which carry the recognition site for the PRE are thus deleted and can be removed from the reaction if desired. Blunt ends are generated at the ends of the fusion product thus providing the recombination site. The blunt ends can for example be directly generated by the use of a PRE restriction enzyme creating blunt ends or by blunting, as for example by fill-in reaction of a cohesive-end or by cleavage of the single-stranded sections by the use of a single-strand specific exonuclease. In a next step the blunt ends are ligated to each other, thus forming a circular recombination product. The circular recombination product is resolved by the use of at least one restriction enzyme recognising a recognition site in the fusion section introduced by the oligonucleotide primers A and/or D.

This embodiment allows the efficient ordered recombination of polynucleotides and/or fragments thereof without a need for homology at the recombination site. Due to the creation of the fusion product head-to-head or tail-to-tail ligated constructs can be avoided, increasing the generation of correctly recombined molecules. Also frameshifts or changes in the amino acid sequence can be avoided due to the use of the PRE since the recombination site can be chosen precisely and in advance. This embodiment is not only useful in recombination processes but for any process involving ligation as for example for the assembly of constructs as vector constructs since the desired fragments can be assembled in an ordered blockwise fashion without the need for homology or restriction sites within the sequence at the assembly site.

There are several possible variant protocols for producing the fusion product. According to one embodiment oligonucleotide primers A and D can be used which carry a recognition site for a restriction enzyme X. Upon cleavage of the amplification products with the restriction enzyme X a matching cohesive end is created at the designated fusion site of each amplification product. Upon ligation of the restriction fragments the fusion product is formed. It is preferred that the restriction enzyme X creates a non-palindromic cohesive end. This feature allows the perfectly ordered ligation of the fragments even though the fragments are ligated at the blunt ends in the next step and preventing rejects caused by head-to-head or tail-to-tail ligation. Due to this feature, the drawbacks known in the State of the Art, concerning blunt ligation wherein often head-to-head or tail-to-tail lligated products are formed, are avoided. It is advantageous to use a PRE as restriction enzyme X.

If it is desired to recombine different fragments in parallel but independent from each other, this can be achieved by ensuring that upon cleavage with the enzyme X unique cohesive ends are created which differ between the groups of fragments not desired to be recombined. Thus recombination of different groups of polynucleotides or fragments thereof can be performed in parallel in one reaction without the danger of unwanted crossovers since the non-matching cohesive ends cannot ligate with each other.

An alternative way to create the fusion product during the amplification process involves the use of two oligonucleotide primers A and D which share complementary sequences at the 5' end. As a result of the amplification the fusion product is directly created. If the complementary sequence is chosen carefully and is for example non-palindromic, it is ensured that only correctly orientated fusion products are created thus enhancing the recombination efficiency and therefore the creation of functional recombinants. If independent recombination of specific groups is desired it is advantageous to use different complementary sequences for polynucleotides, or fragments thereof, which are not desired to recombine with each other.

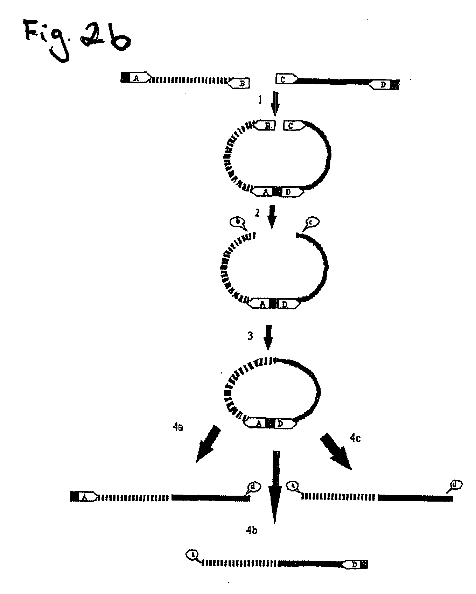

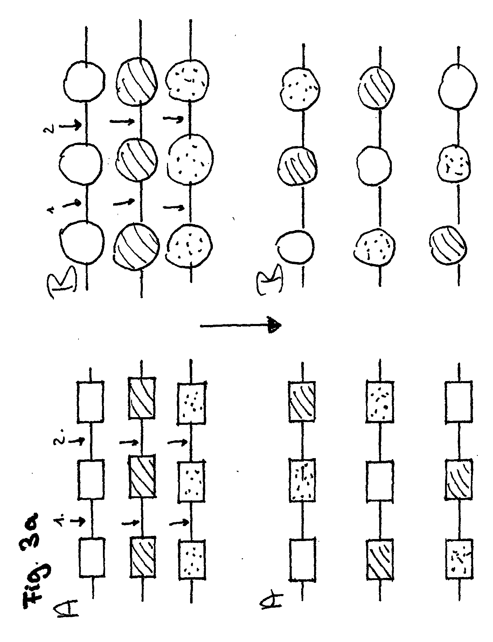

It is advantageous to resolve the circular recombination product by the use of at least one PRE recognising at least one recognition site in the sequence section introduced by the oligonucleotide primers A and D (see Figure 2). This embodiment is preferred if no appropriate restriction sites are available in the parental sequence, since the use of a PRE avoids that additional or changed bases remain in the recombination product, since the recognition site for the PRE is adjacent to the restriction site and is cleaved off during restriction. Thus the oligonucleotide primers can be designed such that the at least one recognition site is positioned for restriction occurring at the end of the fragment sequence leaving no additional bases after cleavage. If appropriate restriction sites are present, or if it is not of disadvantage, other restriction enzymes can be used, as for example, in order to directly clone the recombined fragments into an appropriate expression vector if desired. Also the restriction enzyme X can be used as resolving enzyme.

The process according to the invention also allows selective recombination of individual partial DNA sequences from different libraries. If these are differently degenerated libraries the fragments can be selected in a fashion to maintain structurally or functionally essential elements or schematas while allowing maximal diversity in other fragments. In developing an efficient recombination method it is advantageous that important structural motifs which may have been selected for or which have accumulated during selection rounds are not disrupted. This problem is present in the state of the art (Joern et al., 2002). The so called "schema disruption" describes the extent to which a cross-over disrupts beneficial sequences analogous to its use in computer science and optimisation of genetic algorithms. Also nature shows this principal in order to increase the rate of evolution by allowing higher recombination frequencies in between genomic regions encoding functional or structural domains (exons), i.e. recombination taking place within the long intron encoding regions. This hypothesis is supported by recent data (Voigt et al., 2002).

Thus according to a further embodiment of the present invention it is possible to recombine structural units by introducing the recombination sites to the polynucleotide sequence through the process of the present invention such that structural units are preserved by for example introducing the recombination site in polynucleotide sections defining interstructural motifs adjacent to the respective structural unit or at the end portions of it. According to this embodiment the disruption of structural schemata's as described above can be avoided since beneficial sequences ("schema") are preserved during recombination by introducing the recombination sites at predetermined positions increasing structurally oriented useful or at least correctly folded diversity in the library.

The term structural unit should be understood in a broad sense. Basically all molecules which are made of structural units wherein an ordered structure needs to be maintained can be recombined within the teachings of the invention preserving the overall structure and thus the scaffold of the molecules if desired. For the purpose of this invention the term "structural unit" comprises any selected polynucleotide region with any functional property on any molecular level as e.g. primary, secondary, tertiary or superstructure or domain or interdomain properties and includes for example polynucleotide sections encoding proteins, protein sub-units, protein domains, polypeptide fragments or polypeptide secondary or super secondary structures. The term structural unit also includes the definition of "schema" by Joern et al., 2002 which is fully incorporated by reference or even hypothetical or empirical selections. The actual content of the broad definition of "structural unit" for the present invention will depend on the knowledge available to the person skilled in the art when applying the teaching of the invention, with respect to what motifs, structural elements or selected subgroups are functionally relevant. Also a structural unit can e.g. comprise a polynucleotide section with special characteristics as for example regulatory regions such as promotors, replication origins and RNA processing signals. Also polynucleotide regions defining 3-dimensional structures or sections as for example in aptamers or ribozymes are comprised by this definition and can be recombined with the process according to the present invention.

Due to the freedom to introduce recombination sites, at literally any position in a polynucleotide, it is possible to preserve overall folding structures without being limited to recombination between fragments which share a high homology on the polynucleotide level at the recombination site. Due to the highly efficient cloning process of the present invention a large number of correctly assembled intermediate recombinants can be produced to meet the requirements for high diversity in a library. Further details of this teaching is contained in the European Patent application EP 02016743.3 which is herewith fully incorporated by reference. The recombination methods and processes described in the cited application can be performed very efficiently with the recombination method of the present invention.

It is possible to exchange or assemble polynucleotides or fragments thereof defining structural units in order to evolve for example new functions in proteins by for example redesigning existing protein frameworks with the recombination process according to the present invention. It is also possible to create novel proteins as for example enzymes with desired properties. The method is very efficient in order to evolve molecules from for example variant gene families. Many, especially eukaryotic proteins depict a modular structure wherein the modules (structural units, usually domains) fold independently and can therefore be shuffled without destroying the fold of the module. Examples of such "mobile" domains which can also be shuffled between different proteins are disclosed in Kolkmann and Stemmer, 2001 which is incorporated herewith by reference. The recombination of the present invention can be used in order to evolve multi-domain proteins, to introduce allosteric regulation, to create activators or inhibitors, to improve stability or expression, to modify substrate specificity, to improve the catalytic efficiency, to alter multimodular syntetases, to improve therapeutic properties, to create molecular biosensors and to create novel enzymes as for example chimeric restriction endonucleases. Possible applications of the present invention are disclosed in Ostermeier and Benkovic (2001) as well the examples for alteration of biocatalysts, in particular where directed evolution is mentioned in Burton et al. (2002) both of which are incorporated herewith fully by reference.

According to a further embodiment of the present invention a process for evolving a molecule by selection and recombination is provided which comprises a recombination process as outlined above. The process comprises the creation of a starting library, the recombination of the library members according to the recombination process as outlined above, the selection of candidates with desired characteristics and optionally the performance of further rounds of selection and recombination if desired.

Optionally the polynucleotides are mutagenised before or after a selection step in order to create additional diversity. It is also possible to mutagenise the polynucleotides before the starting library is created. It is even possible to start the population from one parental polynucleotide which is mutagenised in order to create variants. Conventional mutagenesis methods such as for example error-prone PCR, cassette mutagenesis and chemical mutagenesis methods may be performed to.

Preferably the polynucleotides are expressed in a host system in order to select the expression products for their characteristics. The library can be, for example, an expression library wherein the polynucleotides are contained in an expression vector or the polynucleotide itself is a vector construct. It is also possible to clone the recombination products into an appropriate expression vector. The polynucleotides are expressed in a host as for example a cell. Preferably the polynucleotides are expressed such that it is possible to link the genotype to the phenotype. The recombined polynucleotides are expressed in the host and selection is performed by screening or selecting for a particular phenotype among the cell clones as for example whether they can deliver a certain resistance to the host. The expression products can also be screened for a certain characteristics as for example a catalytic activity or a certain affinity.

Various expression systems are known and applicable, including systems comprising: prokaryotes (e.g. E.coli) transformed with, e.g., recombinant phage, phagemid, plasmid or cosmid expression vectors; eukaryotic systems as for example yeast transformed with, for example, yeast expression vectors; insect cell systems transformed with, for example, viral expression vectors; plant cell systems transfected with, for example, viral or bacterial expression vectors; animal cell systems (including human cells) transfected with, for example, adenovirus expression vectors.

The expression vectors may include a prokaryotic replicon, an appropriate promoter capable of directing expression of the genes in the respective host cell. Typical prokaryotic vector plasmids are: pUC18, pUC19, pBR322 and pBR329 available from Biorad laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA). A typical mammalian cell vector is pSVL available from Pharmacia (Piscataway, NJ, USA). Useful yeast plasmid vectors are for example pRS403-406 and pRS413-416 and are available from Stratagene Cloning Systems (La Jolla, CA 92037, USA). Plasmids pRS403, pRS404, pRS405 and pRS406 are Yeast Integrating plasmids (Yips) and incorporate yeast selectable markers. Plasmids pRS413-416 are Yeast Centromere plasmids (Ycps).

In one embodiment the selection procedure comprises the selection for affinity to a defined target. In order to evolve molecules with certain binding characteristics it is preferred to create a display library. The molecules that are displayed on the surface of the host can be easily screened for their binding characteristics by contacting them to the target molecule which is for example immobilised on a carrier. A phage or phagemid library is preferred because of the technical advantages of this vector system as to be outlined below.

The display of proteins or polypeptides on the surface of bacteriophages fused to one of the phage coat proteins as for example pIII or pVIII, provides a powerful tool for the selection of specific ligands. First a polynucleotide encoding a protein or polypeptide for display is cloned into a phage, wherein the cloned polynucleotide is expressed fused to the coat-anchoring part of one of the phage coat proteins (pIII or pVIII in case of a filamentous phage) such that the foreign protein or polypeptide is displayed on the surface of the phage. The phage displaying the protein or polypeptide with the desired properties is then selected (e.g. by affinity methods) thereby providing a genotype (linked to a phenotype) that can be sequenced, multiplied and transferred to other expression systems.

Alternatively, the foreign protein or polypeptide may be expressed using a phagemid vector that can be packaged as a single stranded nucleic acid in a bacteriophage coat. When phagemid vectors are employed a "helper phage" is used which supplies the functions of replication and packaging of the phagemid nucleic acid.

Several methods of selecting phage expressing a protein or peptide with a desired specificity are known in the state of the art and applicable. A widely used method is the so called panning, in which phage stocks displaying ligands are exposed to solid phase coupled target molecules, e.g. using affinity selection. Any method for selecting phages may be used with the present invention.

The use of phage display to isolate ligands that bind biologically relevant molecules is known (Katz (1997) Annual Rev. Biophy. Biomol. Struct. 26, 27-45and Hoogenboom et al. (1998) Immunotechnology 4(1), 1-20). Also proteins and multimeric proteins have been successfully phage displayed (EP 0349578A; EP 0527839A; EP 0589877A; Chiswell and McCafferty, 1992, Trends Biotechnol. 10, 80-84). In addition functional antibody fragments have been expressed (see above). Further information on the principles and practice of phage display is provided in *Phage display of peptides and pro teins: a laboratory manual* by Eds.:. Kay, Winter and McCafferty (1996) Academic Press, Inc ISBN 0-12-402380-0, the disclosure of which is incorporated herein fully by reference.

The generated evolved sequences are expressed in a host cell and can be recovered and purified from recombinant cell cultures by well-known techniques including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. Also high performance liquid chromatography (HPLC) can be employed for purification.

Further a method for producing a molecule with desired characteristics is provided which comprises
- obtaining by the described method a polynucleotide construct encoding or comprising a molecule with the desired characteristics,
- producing either the polynucleotide with the desired characteristics, the expression product having the desired characteristics or a molecule having essentially the same characteristics. The term "molecule having essentially the same characteristics" means for example that the molecule has the same binding domain as the expression product isolated by the process or that a polynucleotide is produced which comprises a sequence section which was evolved by the above process.

The invention will be explained in further detail according to the figures.
- Fig. 1: shows the recombination process according to the invention according to a first embodiment;
- Fig. 2a and b: show the recombination process according to the embodiment involving blunt-end ligation;
- Fig. 3a and 3b: show further embodiments of the present invention;
- Fig. 4: shows a scheme illustrating the generation of an evolved molecule according to one embodiment;
- Fig. 5: shows another scheme illustrating the evolution of a gene according to an embodiment of the present invention;
- Fig. 6: shows a picture of an agarose gel (see example)

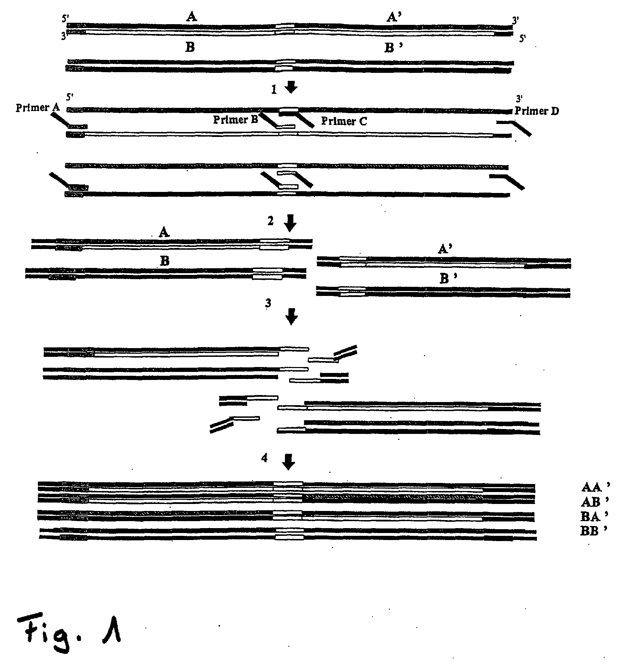

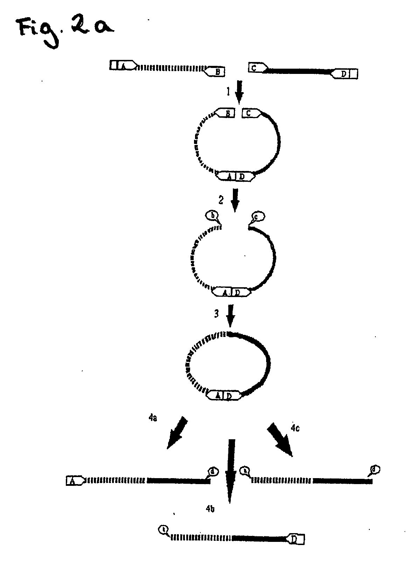

Figure 1 shows two linear double stranded polynucleotide molecules (AA' and variant BB') which are recombined as shown. This scheme should be understood as symbolic for a process intended to be carried out with a large mixture of variant fragments.

In step 1 the polynucleotides are denatured in the presence of four different oligonucleotide primers (primers A, B, C and D) which prime at the end of the polynucleotide fragments, which are supposed to participate in the recombination process and at the desired recombination site. The oligonulceotide primers which prime at the chosen recombination sites carry a recognition site for a PRE at their 5' end. Optionally the oligonucleotide primers priming at the respective ends of the polynucleotides can also carry a recognition site for a restriction enzyme, which can also be a PRE. Those sites can for example be used to provide appropriate cohesive, preferably non-palindromic, ends in order to insert the recombined polynucleotides in an appropriate expression vector or in order to ligate recombined fragments to each other in order to assemble new, longer polynucleotides.

The polynucleotide molecules are subjected to polymerase chain reaction amplification by cycles of denaturation and renaturation in the presence of a heat stable DNA polymerase and oligonucleotide triphosphate. The main amplification products resulting from Step 2 represent the left (A and B) and the right (A' and B') fragments of the molecules. The double stranded DNA sections introduced by the oligonucleotide primers carry a recognition site for a PRE. Thus they are able to bind a PRE which cleaves in the adjacent polynucleotide sequence. Upon cleavage (Step 3) a cohesive end is generated at the desired recombination site which is according to the example a non-palindromic cohesive-end defining the recombination site. The proportion of parental molecules which might survive Steps 1 and 2, can be lowered with successive rounds of PRE amplification in the presence of an excess of internal primers. The small terminal fragments generated by cleavage with the PRE carrying the PRE binding site are preferably removed from the reaction. If the primers are biotinylated they can be removed by binding to streptavidin or avidin coated beads. Alternatively they can be removed by gel purification as it is common knowledge in the state of the art.

The cleaved DNA fragments are ligated together in step 4 with a DNA ligase to produce recombinant (AB', BA') molecules. According to this embodiment very few parental molecules (AA' and BB') will be generated if the initial diversity is very large, i.e. the initial library comprises a large number of variants.

Since the primers were chosen to produce non-palindromic cohesive ends upon cleavage by the PRE, the correct orientation of the fragments is preserved throughout the procedure thus increasing the efficiency of the recombination process according to the invention, since the generation of molecules AB and A'B' is avoided. According to this embodiment already little homology (1 to 8 bases) at the recombination site is sufficient for an efficient recombination.

Figure 2a is a schematic representation wherein two polynucleotide fragments are recombined and the products are confined to ordered oriented recombination products. This scheme should be understood as representing a process intended to be carried out with a large mixture of variant fragments. Two polynucleotide fragments (solid and broken lines) are shown. These may represent actual DNA fragments, single or double stranded, of just a DNA sequence present within longer DNA constructs, linear or circular. Four oligonucleotide primers (A,B,C and D) are provided to promote specific amplification of the shown two variant DNA fragments. The open regions in the primers A and D represent binding sites for a restriction enzyme X that will create non-palindromic but complementary cohesive ends and is preferably a PRE. All primers contain PRE binding sites according to this example, for example typlls restriction endonuclease binding sites.

First the DNA fragments are amplified with the indicated primers. The ends A and D are cleaved with the restriction enzyme X thus creating according to this example non-palindromic but complementary cohesive ends. The cleavage products are ligated together thus forming after step 1 a linear fusion product, wherein the head of the fragment with the broken line is ligated to the tail of the other DNA fragment. Due to the preferred use of a PRE creating non-palindromic cohesive ends as enzyme X the desired ordered assembly is ensured and basically no incorrectly fused constructs are created since the creation of head-to-head and tail-to-tail ligated products which would increase the rate of rejects are avoided. The extensions introduced by the primers B and C are released in step 2 to yield blunt ends at "b" and "c" upon cleavage with the appropriate PRE binding to the recognition site introduced by the primers B and C wherein the blunt ends can be ligated to each other. Ring closure will occur in the absence of formation of concatemers by ligation in step 3 at a relatively low DNA concentration (e.g. < 50µg/ml) thus forming the circular recombination product. Linear DNA products for further ordered manipulation or for selection can be produced by cleavage with the PREs which cleave A and D either simultaneously or consecutively (step 4a to 4c). In particular these products can be inserted in an appropriate library or vector. As will be understood by a person skilled in the art, for this embodiment not even a single base homology is required at the recombination site.

Fig. 2b is a modification of Fig.2a wherein the fusion product is not created by the use of the restriction enzyme X. According to this embodiment the hatched boxes represent complementary sequences at the 5'ends of the PCR oligonucleotide primers A and D. In step 1 all four oligonucleotide primers are present in the reaction and the fusion product is directly created. The other steps are as explained in connection with Fig.2a.

The embodiment of the present invention described with Fig. 2a and 2b is also very useful for a blockwise assembly of constructs as for example vector constructs. According to a slightly modified embodiment it is not necessary that the oligonucleotide primers priming at the desired recombination or assembly site carry a PRE recognition site. This is for example the case when a linker sequence section may be present between the assembled fragments or if a few additional preferably in frame bases or changed bases are not of disadvantage. Due to the creation of the fusion products by the use of a PRE as enzyme X or by the use of a non-palindromic complementary sequence at the 5' end of the primers it is ensured that no head-to head or tail to tail ligated products are created.

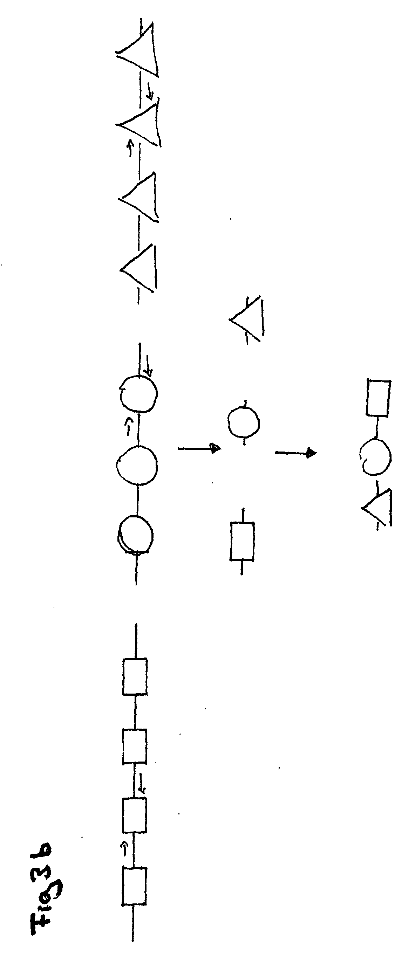

Fig. 3a is a schematic representation of the recombination between different subsets of polynucleotides (group A and group B) present in one reaction tube. The rectangular and round symbols represent the polynucleotide sections which are to be recombined. The small arrows 1 and 2 indicate the recombination site which is generated as outlined above. According to this embodiment different non-palindromic cohesive ends are generated between the subsets A and B preventing the recombination of fragments of group A with fragments of group B. If the non-palindromic cohesive ends generated also differ at the recombination positions 1 and 2 it is ensured that the fragments reassemble in the correct order preventing the formation of rejects.

Fig.3b is a schematic view of the use of the recombination method of the present invention in order to create new polynucleotides respective proteins. The rectangular, triangular and round symbols represent structural units which are to be assembled into a new polynucleotide construct. The small arrows indicate the priming position of the oligonucleotide primers. The polynucleotide sections comprising structural units are amplified with the PRE carrying oligonucleotide primers as described above. The amplified fragments which are to be assembled are cleaved with the respective PRE and ligated. By carefully choosing the non-palindromic cohesive ends comprising the recombination site it is possible to assemble the fragments in an ordered fashion. For example according to the shown example the 3' non-palindromic cohesive end of the triangular fragment was chosen such that it could only ligate with the matching 5' non-palindromic cohesive end of the round fragment while the 3' end of the round symbol only matches the 5' end of the rectangular fragment.

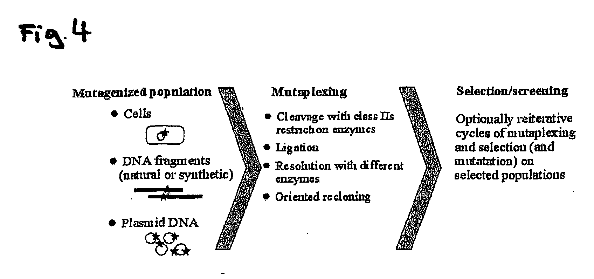

Fig.4 is a scheme illustrating the process for evolving a molecule by selection and recombining according to the present invention. First a mutagenised population is created. This population can for example comprise cells carrying expression vectors, natural or synthetic DNA fragments or vector DNA. The asterisks indicate mutations.

In order to recombine the mutagenised population the DNA is cleaved with a PRE, preferably a type IIS restriction enzyme, creating non-palindromic cohesive ends at the recombination site. The fragments are ligated to each other thus creating recombined molecules. In case circular DNA is created as an intermediate, e.g. as when using the embodiment as shown in Figure 2, the resulting ligation products are cleaved with a resolution enzyme which is preferably a different one than the one used for recombination in order to avoid a re-cleavage of the recombined molecules at the cross-over site. The recombined molecules can be inserted in an appropriate expression vector if the molecules are not already present in a vector.

In a next step the recombined molecules are selected for the desired property. The best clones are selected. Optionally the selected clones can be recombined with each other according to the process of the present invention or further mutated. They can also be backcrossed with the starting library. The cycles of selection and recombination can be performed reiteratively until a molecule is generated which depicts the desired characteristics.

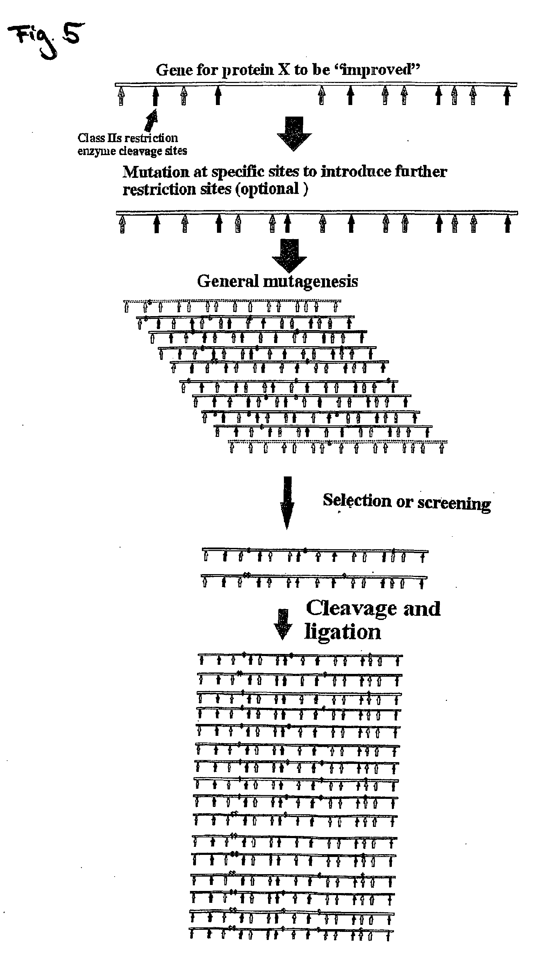

Fig. 5 demonstrates the "improvement" by directed evolution of a gene encoding a protein X according to the invention, i.e. involving incremental selection of desired properties. The arrows represent the desired or present PRE (classlls) cleavage sites which mark the recombination site. The appropriate recognition sites for the PREs can be introduced according to the above explained principle using primers carrying the PRE recognition site at their 5' end. Optionally further recognition respective restriction sites for PRE enzymes can be introduced by site specific mutation. The gene is subjected to a mutagenesis process wherein any mutagenesis process known in the state of the art as for example chemical or UV-induced mutagenesis or error-prone PCR, can be used. Due to the mutagenesis process a population of gene variants are created. This population is screened or selected for gene variants which show improved properties. Those selected candidates are recombined by the use of PRE enzymes thus creating non-palindromic cohesive ends which can be ligated in an ordered fashion thus resulting in a population of correctly recombined molecules. This recombined population is again screened or selected for the best candidates. Optionally reiterative rounds of selection and recombination are performed. It is also possible to additionally mutate the improved population.

### Example

### Recombination of mutated PAI-1 gene fragments

According to this embodiment of the invention a collection of human plasminogen activator inhibitor-I (PAI-I) fragments differing in several positions in their DNA sequence (a collection of mutated gene fragments) is subjected to the recombination process according to the invention and a phagemid-display library thereof is created for further use. It was shown that recombinants are formed with high efficiency.

### Embodiment: efficient recombination of mutated PAI-1 gene fragments

### 1. PCR reactions

For each of the PAI clones A, B and C, 3 PCR reactions were carried out:
PCR1: primer *pai-up* + *pai-bsg-1r* =>PAI fragment 1(465bp)
PCR2: primer *pai-bsg-2f* + *pai bsg-2r* =>PAI fragment 2 (544bp)
PCR3: primer pai-bsg-3f + pai-down =>PAI fragment 3 (287 bp)

### Primer sequences:

pai-up
5'CCCGCTAGCTGGTTTCGCTACCGTAGCGCAGGCCGGCGTGCACCATCCCCCATCCTA 3'

pai-bsg-1r
5' GAGAGGTGCAGCCAGTCATTGATGATGAATCT 3'

pai-bsg-2f
5'     GAGAGGTGCAGGAGAGAGCCAGATTCATCAT     3'

pai_down
5'    GGGGGTACCCTGTACTAGTGGGTTCCATCACTTGGCCCATGAA    3'

pai-bsg-2r
5'      GAGAGGTGCAGCCTGAAACTGTCTGAACATG   3'

pai-bsg-3f
5' GAGAGGTGCAGGAATGACCGACATGTTCAGA 3'

50µl PCR assay:
primers 0,5µM each
plasmid-DNA 5ng
dNTPs 200µM
Vent polymerase (NEB) 2U
1x polymerase buffer (NEB)
hot start: addition of polymerase at 95°C
PCR program: 2min 95°C/ (30s 95°C / 30s 60°C / 45s 72°C) 30x

### 2. Purification of PCR products

PCR products were purified using glass fiber columns (GFX^{TM} PCR DNA and Gel Band Purification Kit, Amersham Pharmacia). DNA was eluted with 50µl TE buffer (10 mM Tris, pH 8.0, 0.1 mM EDTA).

### 3. BsgI digestion of PCR products

50µl assay:
ca. 5µg purified PCR product
SAM 100µM
1x NEB4 buffer
BsgI (NEB) 6U
3h 37°C

### 4. Purification of BsgI digested PCR products

GFX columns (see above), elution with 40µl TE buffer (see above).

### 5. Ligation

approach 1: equal mass from the 9 different PCR fragments were added using two different concentrations (1a and 1b)
approach 2: equal molar concentrations of the 9 different PCR fragments were added using two different concentrations (2a and 2b)

120µl ligation assay:
12U T4-Ligase
**BsgI digested and purified PCR fragments 1, 2 and 3 of PAI clones A, B and C in different amounts (see below*)**
inkubation o.n. 10°C
Ligase inactivation 20 min 65°C

*
   **1a:** 12µl of each fragment
   **1b:** 3µl of each fragment
   **2a:**
      fragment1: 24µl
      fragment2 : 6,9µl
      fragment3: 12µl
   2b:
      fragment1: 6µl
      fragment2: 27,6µl
      fragment3: 48µl

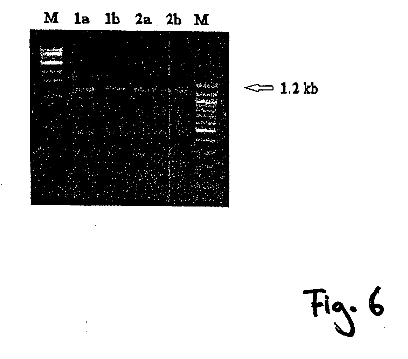

**Figure 6:** Ligation products 1a, 1b ,2a, 2b were separated by agarose gel electrophoresis (M = molecular weight marker). The main product, shown with an arrow (1,2kb band), represents the full length PAI gene.

### 6. Extraction of 1,2kb fragment from preparative agarose gel

20µl inactivated ligation assay 2a (see above) was separated by preparative ararose gel electrophoresis. The 1,2kb band (corresponding to the size of PAI gene) was excised, dissolved in 50µl TE buffer and incubated for 5 min at 60°C. The sample was centrifuged in a microcentrifuge for 10min at 13000rpm and the supernatant was used for the subsequent PCR reaction.

### 7. Amplification of 1,2 kb fragment by PCR

The 1, 2kb fragment extracted from agarose gel was amplified by PCR using end primers *pai-up* and *pai-down*
100µl assay:
Primers 0,5µM each
5µl extracted DNA (see above)
dNTPs 200µM
Vent polymerase (NEB) 2U
1x polymerase buffer (NEB)
hot start: addition of polymerase at 95°C
PCR program: 2min 95°C/ (30s 95°C / 30s 48°C / 90s 72°C) 30x

### 8. Purification of PCR products

GFX columns, elution with 40µl TE buffer.

### 9. NheI und KpnI digestion of purified PCR products

100µl assay:
20µl purified PCR products (ca 2µg DNA)
10U NheI, 10U KpnI
1x buffer NEB1 + BSA
2,5h 37°C, enzyme inactivation 20 min at 65°C

### 10. Purification of NheI/KpnI digested PCR products

GFX columns, elution with 50µl TE buffer.

### 11. Preparation of the PAI vector "cp14am25"

### NheI/KpnI digestion

200µl assay:
10µg PAI vector ("cp14am25")
20U NheI, 20U KpnI
buffer NEB 1+ BSA
2,5h 37°C

### Purification from gel band

4,5kb fragment was purified from gel band (GFX^{TM} PCR DNA and Gel Band Purification Kit, Amersham Pharmacia) , DNA was eluted with 100µl TE buffer.

### Dephosphorylation

50µl assay:
30µl NheI/KpnI digested and purified PAI vector (ca 3,5µg DNA)
CIAP (calf intestine alcaline phosphatase, 0,06U)
1x CIAP buffer
30 min 37°C, enzyme inactivation 20min 85°C

### 12. Ligation

4 approaches were carried out using different vector/insert ratios and different DNA concentrations
1. molar ratio vector/insert 1:3,3
   1µl vector dephosphorylated (60ng)
   1,7µl insert (50ng)
   1a. 20µl assay
   1b. 100µl assay
2. molar ratio vector/insert 1:6,6
   1ml vector dephosphorylated (60ng)
   3,3µl insert (50ng)
   2a. 20ml assay
   2b. 100µl assay
3. control without insert
   1µl vector dephosphorylated (60ng)
   3a. 20µl assay
   3b. 100µl assay
   After ligation for 1h at room temperature (T4 ligase 1U/10µl in 1x ligase buffer), the enzyme was inactivated for 15min at 65°C.

### 13. Transformation in E. coli JM101λ.

Ligation products were transformed in *E.coli* JM101λ, by electroporation.
Number of clones per transformation of 5µl DNA:
1a: 6,5x 10³
1b: 1,3x 10³
2a: 2,8x 10⁴
2b: 3,8x 10³
3a: 3x 10²
3b: 1x 10²

### 14. Colony PCR

25µl PCR assay:
DNA: 5 µl of a colony lysis solution (single colony resuspended in RNase buffer (50mM Tris/HCl pH7,5, 10mM EDTA, 100µg/ml RNAse A) and heated for 3 min at 95°C)
primer Claus21 (ACAACTTGCTTGGATTCCTA) 0,8µM
primer gen301 (CTTTCCAGACGTTAGTAAATG) 0,8 µM
dNTPs 240µM
Vent polymerase (NEB) 0,5U
1x polymerase buffer (NEB)
hot start: addition of dNTPs and polymerase at 95°C
PCR program: 2min 95°C / (30s 95°C/ 30s 48°C/ 90s 72°C) 35x

### 15. DNA sequencing of colony PCR products

### Data on products obtained after mutaplexing: Mutaplexing based on PCR fragments

In order to demonstrate the method of mutaplexing based on PCR fragments the PAI gene was amplified by PCR in three fragments. The PCR primers contained a recognition site for a typeIIS restriction enzyme (BsgI) at their 5'-end that allowed the digestion and subsequent ligation of the PCR fragments.
The PCR reactions were carried out separately for three PAI clones (A, B and C) having different mutations (see table 1) . After ligation of the 9 PCR fragments recombination could be proved by DNA sequencing of resulting clones.

**Table 1:**

| Mutations of PAI clones A, B und C | | | |
|---|---|---|---|
| Clone | mutations fragment1 (1-432) | mutations fragment2 (433-927) | mutations fragment3 (828-1207) |
| A | A1 (a/g78) A2 (c/t166) A3 (a/g216) A4 (c/t304) | A5 (g/c715) A6(g/a793) A7(c/g884) | A8 (c/t992) A9(a/g1004) A10(g/a1055) |
| B | B1 (t/c64) B2(g/a111) B3 (c/t384) | B4 (a/g489) B5(a/g656) B6 (c/t690) B7 (c/g714) | - |
| C | C1 c/a203) C2 (c/t271) | C3 (a/t487) C4 (t/g497) C5 (g/c507) C6 (c/t722) C7 (c/t746) C8 (t/a911) | C9 (a/g1091) |

Following steps were performed as described:
1. PCR reactions for PAI clones A, B and C separately
2. Purification of PCR products
3. BsgI digestion of PCR products
4. Purification of Bsgl digested PCR products
5. Test ligations and ligation of PCR products
6. Extraction of 1,2kb product from agarose gel
7. Amplification of 1,2 kb fragment by PCR
8. Purification of PCR products
9. Nhel and Kpnl digestion of PCR products
10. Purification of NheI/KpnI digested PCR products
11. Preparation of the PAI vector (NheI/KpnI digestion, purification, dephosphorylation)
12. Ligation of PCR fragments and PAI vector
13. Transformation in *E. coli* JM101l
14. Colony PCR
15. DNA sequencing of Colony PCRs

### Result

After mutaplexing with PCR fragments the DNA sequences of 24 PAI clones were determined. The origin of the three fragments of resulting clones could be analysed by comparison with the DNA sequences of starting clones A, B and C. in table 2 mutations present in clones after mutaplexing are listed. All clones had maintained the order and orientation of the fragments. No truncation nor insertion mutants were detected. It was shown that there is no significant bias with respect to parental origin of the fragments found in the mutaplexed clones (fragment origin: A, 27; B,24; C 33[= equals more than a sum of 72 due to internal recombination]). Further recombination was seen to have occurred within 15 from 72 fragments In 11 from 24 clones.

**Tab.2:**

| Analysis of recombination of PAI clones after mutaplexing An asterisk indicates that recombination has ocurred within the amplified fragements, in addition to recombination at the mutaplexing junction sites. | | | |
|---|---|---|---|
| PAI-Klon | Mutations present (underlining shows parental origin) | | |
| | Fragment1 | Fragment2 | Fragment3 |
| 1 | B1B2B3 | C3C4C5C6C7C8 | A8A9A10 |
| 2 | B1B283 | A5A6A7 | C9 |
| 3* | C1C2 | C3C4C5C6C7*A6A7 | C9 |
| 5 | A1A2A3A4 | C3C4C5C6C7C8 | A8A9A10 |
| **6*** | C1C2 | C3C4C5C6* A6A7 | - |
| 8 | B1B2B3 | C3C4C5C6C7C8 | - |
| 9* | C1C2 | C3C4C5* A5A6A7 | C9 |
| 11 | A1A2A3A4 | C3C4C5C6C7C8 | C9 |
| 28 | B1B2B3 | A5A6A7 | C9 |
| 29 | C1C2 | A5A6A7 | - |
| 30 | C1C2 | A5A6A7 | - |
| **31*** | A1A2*C1C2 | C3C4C5C6*C8 | C9 |
| 32* | B1B2*A2A3A4*B3 | A5A6A7 | C9 |
| **33** | A1A2A3A4 | C3C4C5C6C7C8 | A8A9A10 |
| 34 | B1B2B3 | C3C4C5* B5B6B7 | - |
| **35*** | A1A2A3* C2 | B4BSB6* C6C7C8 | C9 |
| 36 | A1A2A3A4 | C3C4C5C6C7C8 | - |
| 37 | B1B2B3 | A5A6A7 | - |
| 38 | B1B2B3 | A5A6A7 | A8A9A10 |
| 39* | A1A2A3A4 | A5 | A8A9A10* C9 |
| 40* | A1A2A3* | *C6C7C8 | - |
| 41* | A1 C1C2* B3 | *B6B7 | C9 |
| 42* | A2A3 | C3C4C5* B586B7*A7 | - |
| 43* | B2 *C2 | C3C4C5C6C7C8 | C9 |
| Order of mutations: N.B. mutations from:parent A are double-, B not, and C singly underlined: **Fragment1** B1A1B2A2 C1 A3 C2 A4B3 **Fragment2** C3 B4 C4C5B5B6B7A5 C6C7 A6A7 C8 **Fragment3** A8A9A10 C9 | | | |

DNA sequence PAI gene and primers

LeuAlaGlyPheAlaThrValAlaGlnAlaGlyValHisHisProProSerTyrValAla
5' GCTAGCTGGTTTCGCTACCGTAGCGCAGGCCGGCGTGCACCATCCCCCATCCTACGTGGC 3'
CCCGCTAGCTGGTTTCGCTACCGTAGCGCAGGCCGGCGTGCACCATCCCCCATCCTA (pai-up)
NheI 10 20 30 40 50 60
3' CGATCGACCAAAGCGATGGCATCGCGTCCGGCCGCACGTGGTAGGGGGTAGGATGCACCG 5'

HisLeuAlaSerAspPheGlyValArgValPheGlnGlnValAlaGlnAlaSerLysAsp
5' CCACCTGGCCTCAGACTTCGGGGTGAGGGTGTTTCAGCAGGTGGCGCAGGCCTCCAAGGA 3'
70 80 90 100 110 120
3' GGTGGACCGGAGTCTGAAGCCCCACTCCCACAAAGTCGTCCACCGCGTCCGGAGGTTCCT 5'

AlaAsnValValPheSerProTyrGlyValAlaSerValLeuAlaMetLeuGlnLeuThr
5' CGCGAACGTGGTTTTCTCACCCTATGGGGTGGCCTCGGTGTTGGCCATGCTCCAGCTGAC 3'
130 140 150 160 170 180
3' GCGCTTGCACCAAAAGAGTGGGATACCCCACCGGAGCCACAACCGGTACGAGGTCGACTG 5'

ThrGlyValGluThrGlnGlnGlnIleGlnAlaAlaMetGlyPheLysIleAspAspLys
5' AACAGGAGTAGAAACCCAGCAGCAGATTCAAGCAGCTATGGGATTCAAGATTGATGACAA 3'
190 200 210 220 230 240
3' TTGTCCTCATCTTTGGGTCGTCGTCTAAGTTCGTCGATACCCTAAGTTCTAACTACTGTT 5'

GlyMetAlaProAlaLeuArgHisLeuTyrLysGluLeuMetGlyProTrpAsnLysAsp
5' GGGCATGGCCCCCGCCCTCCGGCATCTGTACAAGGAGCTCATGGGGCCATGGAACAAGGA 3'
250 260 270 280 290 300
3' CCCGTACCGGGGGCGGGAGGCCGTAGACATGTTCCTCGAGTACCCCGGTACCTTGTTCCT 5'

GluIleSerThrThrAspAlaIlePheValGlnArgAspLeuLysLeuValGlnGlyPhe
5' CGAGATCAGCACCACAGACGCGATCTTCGTCCAGCGGGATCTGAAGCTGGTCCAGGGCTT 3'
310 320 330 340 350 360
3' GCTCTAGTCGTGGTGTCTGCGCTAGAAGCAGGTCGCCCTAGACTTCGACCAGGTCCCGAA 5'

MetProHisPhePheLysLeuPheArgSerThrValLysGlnValAspPheSerGluVal
5' CATGCCCCACTTCTTCAAGCTGTTCCGGAGCACGGTCAAGCAAGTGGACTTTTCAGAGGT 3'
GAGAGGTGCA
370 380 390 400 410 420
3' GTACGGGGTGAAGAAGTTCGACAAGGCCTCGTGCCAGTTCGTTCACCTGAAAAGTCTCCA 5'

GluArgAlaArgPheIleIleAsnAspTrpValLysThrHisThrLysGlyMetIleSer
5' GGAGAGAGCCAGATTCATCATCAATGACTGGGTGAAGACACACACAAAAGGTATGATCAG 3'
GGAGAGAGCCAGATTCATCAT (pai-bsg-2f)
430 440 450 460 470 480
3' CCTCTCTCGGTCTAAGTAGTAGTTACTGACCCACTTCTGTGTGTGTTTTCCATACTAGTC 5'
TCTAAGTAGTAGTTACTGACCGACGTGGAGAG (pai-bsg-1r)

AsnLeuLeuGlyLysGlyAlaValAspGlnLeuThrArgLeuValLeuValAsnAlaLeu
5' CAACTTGCTTGGGAAAGGAGCCGTGGACCAGCTGACACGGCTGGTGCTGGTGAATGCCCT 3'
490 500 510 520 530 540
3' GTTGAACGAACCCTTTCCTCGGCACCTGGTCGACTGTGCCGACCACGACCACTTACGGGA 5'

TyrPheAsnGlyGlnTrpLysThrProPheProAspSerSerThrHisArgArgLeuPhe
5' CTACTTCAACGGCCAGTGGAAGACTCCCTTCCCCGACTCCAGCACCCACCGCCGCCTCTT 3'
550 560 570 580 590 600
3' GATGAAGTTGCCGGTCACCTTCTGAGGGAAGGGGCTGAGGTCGTGGGTGGCGGCGGAGAA 5'

HisLysSerAspGlySerThrValSerValProMetMetAlaGlnThrAsnLysPheAsn
5' CCACAAATCAGACGGCAGCACTGTCTCTGTGCCCATGATGGCTCAGACCAACAAGTTCAA 3'
610 620 630 640 650 660
3' GGTGTTTAGTCTGCCGTCGTGACAGAGACACGGGTACTACCGAGTCTGGTTGTTCAAGTT 5'

TyrThrGluPheThrThrProAspGlyHisTyrTyrAspIleLeuGluLeuProTyrHis
5' CTATACTGAGTTCACCACGCCCGATGGCCATTACTACGACATCCTGGAACTGCCCTACCA 3'
670 680 690 700 710 720
3' GATATGACTCAAGTGGTGCGGGCTACCGGTAATGATGCTGTAGGACCTTGACGGGATGGT 5'

GlyAspThrLeuSerMetPheIleAlaAlaProTyrGluLysGluValProLeuSerAla
5' CGGGGACACCCTCAGCATGTTCATTGCTGCCCCTTATGAAAAAGAGGTGCCTCTCTCTGC 3'
730 740 750 760 770 780
3' GCCCCTGTGGGAGTCGTACAAGTAACGACGGGGAATACTTTTTCTCCACGGAGAGAGACG 5'

LeuThrAsnIleLeuSerAlaHisValMetSerHisTrpLysGlyAsnMetThrArgLeu
5' CCTCACCAACATTCTGAGTGCCCACGTCATGAGCCACTGGAAAGGCAACATGACCAGGCT 3'
790 800 810 820 830 840
3' GGAGTGGTTGTAAGACTCACGGGTGCAGTACTCGGTGACCTTTCCGTTGTACTGGTCCGA 5'

ProArgLeuLeuValLeuProLysPheSerLeuGluThrGluValAspLeuArgLysPro
5' GCCCCGCCTCCTGGTTCTGCCCAAGTTCTCCCTGGAGACTGAAGTCGACCTCAGGAAGCC 3'
850 860 870 880 890 900
3' CGGGGCGGAGGACCAAGACGGGTTCAAGAGGGACCTCTGACTTCAGCTGGAGTCCTTCGG 5'

LeuGluAsnLeuGlyMetThrAspMetPheArgGlnPheGlnAlaAspPheThrSerLeu
5' CCTAGAGAACCTGGGAATGACCGACATGTTCAGACAGTTTCAGGCTGACTTCACGAGTCT 3'
GAGAGGTGCAGGAATGACCGACATGTTCAGA (pai-bsg-3f)
910 920 930 940 950 960
3' GGATCTCTTGGACCCTTACTGGCTGTACAAGTCTGTCAAAGTCCGACTGAAGTGCTCAGA 5'
(pai-bsg-2r) GTACAAGTCTGTCAAAGTCCGACGTGGAGAG

SerAspGlnGluProLeuHisValAlaGlnAlaLeuGlnLysValLysIleGluValAsn
5' TTCAGACCAAGAGCCTCTCCACGTCGCGCAGGCGCTGCAGAAAGTGAAGATCGAGGTGAA 3'
970 980 990 1000 1010 1020
3' AAGTCTGGTTCTCGGAGAGGTGCAGCGCGTCCGCGACGTCTTTCACTTCTAGCTCCACTT 5'

GluSerGlyThrValAlaSerSerSerThrAlaValIleValSerAlaArgMetAlaPro
5' CGAGAGTGGCACGGTGGCCTCCTCATCCACAGCTGTCATAGTCTCAGCCCGCATGGCCCC 3'
1030 1040 1050 1060 1070 1080
3' GCTCTCACCGTGCCACCGGAGGAGTAGGTGTCGACAGTATCAGAGTCGGGCGTACCGGGG 5'

GluGluIleIleMetAspArgProPheLeuPheValValArgHisAsnProThrGlyThr
5' CGAGGAGATCATCATGGACAGACCCTTCCTCTTTGTGGTCCGGCACAACCCCACAGGAAC 3'
1090 1100 1110 1120 1130 1140
3' GCTCCTCTAGTAGTACCTGTCTGGGAAGGAGAAACACCAGGCCGTGTTGGGGTGTCCTTG 5'

ValLeuPheMetGlyGlnValMetGluProThrSerThrGlyTyr
5' AGTCCTTTTCATGGGCCAAGTGATGGAACCCACTAGTACAGGGTACC 3'
1150 1160 1170 1180 KpnI
3' TCAGGAAAAGTACCCGGTTCACTACCTTGGGTGATCATGTCCCATGG 5'
AAGTACCCGGTTCACTACCTTGGGTGATCATGTCCCATGGGGG (pai_down)

Fragment 1 (465 bp), primers:
pai-up
5'CCCGCTAGCTGGTTTCGCTACCGTAGCGCAGGCCGGCGTGCACCATCCCCCATCCTA 3'
pai-bsg-1r
5' GAGAGGTGCAGCCAGTCATTGATGATGAATCT 3'

Fragment 2 (544 bp), primers:
pai-bsg-2f
5' GAGAGGTGCAGGAGAGAGCCAGATTCATCAT 3'
pai-bsg-2r
5' GAGAGGTGCAGCCTGAAACTGTCTGAACATG 3'

Fragment 3 (287 bp), primers:
pai-bsg-3f
5' GAGAGGTGCAGGAATGACCGACATGTTCAGA 3'
pai_down
5' GGGGGTACCCTGTACTAGTGGGTTCCATCACTTGGCCCATGAA 3'

### Reference list:

[1] A. K. Aggarwal, D. A. Wah, Curr. Op. Struct. Biol., 1998, 8, 19-25.
[2] S. G. Burton, D. A. Cowan, J. M. Woodley Nature Biotechnol. , 2002, 20, 37-45.
[3] Coco, W., Levinson, W., Crist, M., Hektor, H., Darzins, A., Oienkos, P., Squires, C., Monticello, D Nature Biotechnol., 2001, 19, 354-359.
[4] J. Collins, Ann. Rep. Combinat. Chem. Molec. Divers., 1997,1, 210-262.
[5] A. Crameri, W. P. C. Stemmer, BioTechniques, 1995, 18.
[6] A. Crameri, E. A. Whitehorn, E. Tate, W. P. C. Stemmer, Nature Biotechnology, 1996, 14, 315-319.
[7] A. Crameri, S.-A. Raillard, E. Bermudez, W. P. C. Stemmer, Nature(Lond.), 1998, 391, 288-92.
[8] K. Deshayes, M. L. Schaffer, N. J. Skelton, G. R. Nakamura, S. Kadkhodayan, S. S. Sidhu, Chemistry & Biology, 2002, 9, 495-505.
[9] J. M. Joern, P. Meinhold, F. H. Arnold, J. Molec. Biol., 2002, 316,634-656.
[10] Kikuchi, M., Ohnishi, K., and Harayama S., Gene 1999, 236,159-167.
[11] J. A. Kolkman, W. P. C. Stemmer, Nature Biotechnol., 2001, 19, 354-359.
[12] H. Murakami, T. Hohsaka and M. Sisido, Nature Biotechnol., 2002, 20, 76-81
[13] M. Ostermeier and S. Benkovic, 2000, Adv Protein Chem, 55, 29-77.
[14] A. J. Podhajska, W. Szybalski, Gene, 1985, 40, 175-182.
[15] C. Schmidt-Dannert, D. Umeno, F. H. Arnold, Nat. Biotechnol., 2000, 18, 750-753.
[16] Z. Shao, H. Zhao, L. Giver, F. H. Arnold, Nucleic Acids Res., 1998, 681-683.
[17] Sieber, V., Martinez, C., Arnold, H, Nature Biotechnol., 2001, 19, 456-460.
[18] W. P. C. Stemmer, BioTechnology, 1995, 13, 549-553.
[19] W. P. C. Stemmer, A. Crameri, K. D. Ha, T. M. Brennan, H. L. Heyneker, Gene, 1995, 164, 49-53.
[20] W.P.C. Stemmer and S.K. Morris, 1992, Biotechniques, 13 (2), 214-220.
[21] W. Szybalski, Gene, 1985, 40, 169-172.
[22] C. A. Voigt, C. Martinez, Z.-G. Wang, S. L. Mayo, F. H. Arnold, Nature Struct. Biol., 2002, 9.
[23] R. A. Katz, Annual Rev. Biophy. Biomol. Struct. , 1997, 26, 27-45.
[24] H. R. Hoogenboom et al., Immunotechnology, 1998, 4(1 ), 1-20.
[25] D. J. Chiswell, J. McCafferty, Trends Biotechnol., 1992, 10, 80-84.
[26] B. J. Kay, G. Winter, J. McCafferty (Eds.) Phage display of peptldes and proteins: a *laboratory manual* (1996) Academic Press, Inc ISBN 0-12-402380-0.
[27] Zhao, H., Giver, L., Shao, Z., and Arnold, F.H., 1998,. Nat. Biotechnology, 16, 258-261.

## Claims

1. A recombination process for recombining at least fragments of variant polynucleotides, wherein at least fragments of the polynucleotides are amplified by the use of at least one oligonucleotide primer which comprises a 5' sequence section carrying a recognition site for a PRE and which primes at the desired recombination site,
the amplification products are cleaved with at least one PRE recognising the recognition site introduced by the at least one oligonucleotide primer, thus creating a recombination site, the cleaved fragments are ligated thus producing recombinant molecules.

2. A process according to claim 1, wherein at least two different oligonucleotide primers are used for amplification wherein both comprise a 5' sequence section carrying a recognition site for a PRE.

3. A process according to claim 1 or 2, wherein the small terminal fragments produced in the cleavage step 3 are removed before ligation.

4. A process according to at least one of the above claims, wherein recombination is performed between variant polynucleotides with little or no homology at the recombination site.

5. A process according to at least one of the above claims, wherein upon restriction non-palindromic cohesive ends are generated at the recombination site.

6. A process according to at least one of the above claims, wherein upon restriction with the PRE blunt ends are created.

7. A process according to at least one of the above claims,
- wherein at least four oligonucleotide primers A, B, C and D are provided for amplification,
- wherein the oligonucleotide primers B and C prime at the desired recombination site and comprise a 5' sequence section carrying a recognition site for a PRE,
- wherein the primers A and D carry at least one recognition site for a restriction enzyme;
- wherein the polynucleotides are amplified
- wherein a linear fusion product comprising the amplification products is created,
- wherein the fusion product is cleaved with at least one PRE recognising the recognition sites in the primers B and C
- wherein blunt ends are generated at the ends of the fusion product
- wherein the blunt ends are ligated to each other thus leading to a circular recombination product
- wherein the circular recombination product is resolved by the use of at least one restriction enzyme recognising a recognition site in the primers A and/or D.

8. A process according to at least one of the above claims, wherein the fusion product is created by
- cleaving the amplification products with a restriction enzyme X which recognises a recognition site present in the primers A and D thus creating a matching cohesive end in each amplification product;
- ligating the restriction fragments.

9. A process according to at least one of the above claims, wherein by cleavage with restriction enzyme X a non-palindromic cohesive end is generated.

10. A process according to at least one of the above claims, wherein the restriction enzyme X is a PRE.

11. A process according to at least one of the above claims, wherein the fusion product is created during the amplification process by the use of two primers A and D which share complementary sequences at their 5' ends.

12. A process according to at least one of the above claims, wherein the circular recombination product is resolved by the use of at least one PRE recognising a recognition site in the sequence section introduced by the primers A and D.

13. A process for evolving a molecule by selection and recombination, comprising a recombination process according to at least one of the claims 1 to 12.

14. A process according to claim 13, comprising:
- creating or choosing a library
- recombining the library members with a process according to at least one of the claims 1 to 12
- selecting candidates with desired characteristics
optionally performing further rounds of selection and recombination.

15. A process according to claim 13 or 14, wherein the recombined polynucleotides are expressed in a host wherein the host is selected from the group of prokaryotes, eukaryotic systems as for example yeast, insect cell systems, plant cell systems, animal cell systems.

16. A process according to at least one of the above claims 13 to 15, wherein the polynucleotides and/or the expression product of the polynucleotides are selected for characteristic properties.

17. A process according to at least one of the above claims 13 to 16, wherein the polynucleotides are mutagenised before and/or after recombination.

18. A process according to at least one of the above claims 13 to 17 wherein further in vitro and/or in vivo recombination processes are performed.

19. A process according to at least one of the above claims 14 to 18, wherein the library is a mutational library starting from a collection of polynucleotide fragments encoding at least structural units of a single heteromeric protein product, preferentially a dimeric product.

20. A process according to at least one of the above claims 14 to 19, wherein the library of polynucleotides is composed of a mutagenised collection of human plasminogen activator inhibitor 1 (PAI-I) cDNA fragments wherein the polynucleotides are expressed and displayed in a phagemid expression system.

21. A process according to at least one of the claims 14 to 20, wherein in a first selection library members having desirable characteristics are preselected and wherein recombination is performed between the subset of preselected candidates.

22. A method for producing a molecule with desired characteristics which comprises
- obtaining by the method of at least one of the claims according to claim 13 to 21 a polynucleotide construct encoding or comprising a molecule with desired characteristics,
- producing either the polynucleotide with the desired characteristics, the expression product having the desired characteristics or a molecule having essentially the same characteristics.


SEQUENCE LISTING

<110>  Cosmix molecular biologicals GmbH

<120>  Recombination process for recombining fragments of variant
 polynucleotides

<130>  44786

<140>  EP02021514.1-2402
<141>  2002-09-26

<150>  EP02020639.7
<151>  2002-09-13

<160>  9

<170>  PatentIn version 3.1

<210>  1
<211>  57
<212>  DNA
<213>  Artificial Sequence

<220>
<223>  primer "pai-up" for mutated plasminogen activator inhibito
r-1 (PA
       I-1) gene fragments

<400>  1
cccgctagct ggtttcgcta ccgtagcgca ggccggcgtg caccatcccc catccta
     57


<210>  2
<211>  32
<212>  DNA
<213>  Artificial Sequence

<220>
<223>  primer "pai-bsg-1r" for mutated plasminogen activator inhi
bitor-1
        (PAI-1) gene fragments

<400>  2
gagaggtgca gccagtcatt gatgatgaat ct
     32


<210>  3

<211>  31
<212>  DNA
<213>  Artificial Sequence

<220>
<223>  primer "pai-bsg-2f" for mutated plasminogen activator inhi
bitor-1
        (PAI-1) gene fragments

<400>  3
gagaggtgca ggagagagcc agattcatca t
     31



<210>  4
<211>  31
<212>  DNA
<213>  Artificial Sequence

<220>
<223>  primer "pai-bsg-2r" for mutated plasminogen activator inhi
bitor-1
        (PAI-1) gene fragments

<400>  4
gagaggtgca gcctgaaact gtctgaacat g
     31



<210>  5
<211>  31
<212>  DNA
<213>  Artificial Sequence

<220>
<223>  primer "pai-bsg-3f" for mutated plasminogen activator inhi
bitor-1
        (PAI-1) gene fragments

<400>  5
gagaggtgca ggaatgaccg acatgttcag a
     31



<210>  6
<211>  43
<212>  DNA
<213>  Artificial Sequence

<220>

<223>  primer "pai_down" for mutated plasminogen activator inhibi
tor-1 (
       PAI-1) gene fragments

<400>  6
gggggtaccc tgtactagtg ggttccatca cttggcccat gaa
     43



<210>  7
<211>  20
<212>  DNA
<213>  Artificial Sequence

<220>
<223>  primer "Claus21" for mutated plasminogen activator inhibit
or-1 (P
       AI-1) gene fragments (colony PCR)

<400>  7
acaacttgct tggattccta
     20



<210>  8
<211>  21
<212>  DNA
<213>  Artificial Sequence

<220>
<223>  primer "gen301" for mutated human plasminogen activator in
hibitor
       -1 (PAI-1) gene fragments (colony PCR)

<400>  8
ctttccagac gttagtaaat g
     21



<210>  9
<211>  1187
<212>  DNA
<213>  Homo sapiens

<400>  9
gctagctggt ttcgctaccg tagcgcaggc cggcgtgcac catcccccat cctacgtggc
     60

ccacctggcc tcagacttcg gggtgagggt gtttcagcag gtggcgcagg cctccaagga
    120

cgcgaacgtg gttttctcac cctatggggt ggcctcggtg ttggccatgc tccagctgac
180
aacaggagta gaaacccagc agcagattca agcagctatg ggattcaaga ttgatgacaa
240
gggcatggcc cccgccctcc ggcatctgta caaggagctc atggggccat ggaacaagga
300
cgagatcagc accacagacg cgatcttcgt ccagcgggat ctgaagctgg tccagggctt
360
catgccccac ttcttcaagc tgttccggag cacggtcaag caagtggact tttcagaggt
420
ggagagagcc agattcatca tcaatgactg ggtgaagaca cacacaaaag gtatgatcag
480
caacttgctt gggaaaggag ccgtggacca gctgacacgg ctggtgctgg tgaatgccct
540
ctacttcaac ggccagtgga agactccctt ccccgactcc agcacccacc gccgcctctt
600
ccacaaatca gacggcagca ctgtctctgt gcccatgatg gctcagacca acaagttcaa
660
ctatactgag ttcaccacgc ccgatggcca ttactacgac atcctggaac tgccctacca
720
cggggacacc ctcagcatgt tcattgctgc cccttatgaa aaagaggtgc ctctctctgc
780
cctcaccaac attctgagtg cccacgtcat gagccactgg aaaggcaaca tgaccaggct
840
gccccgcctc ctggttctgc ccaagttctc cctggagact gaagtcgacc tcaggaagcc
900
cctagagaac ctgggaatga ccgacatgtt cagacagttt caggctgact tcacgagtct
960
ttcagaccaa gagcctctcc acgtcgcgca ggcgctgcag aaagtgaaga tcgaggtgaa
1020
cgagagtggc acggtggcct cctcatccac agctgtcata gtctcagccc gcatggcccc
1080
cgaggagatc atcatggaca gacccttcct ctttgtggtc cggcacaacc ccacaggaac

1140

agtccttttc atgggccaag tgatggaacc cactagtaca gggtacc
1187